# EUROPÄISCHE PATENTSCHRIFT

(11) **EP 3 538 183 B1**
(45) Veröffentlichungstag und Bekanntmachung des Hinweises auf die Patenterteilung: **13.03.2024**
(21) Anmeldenummer: 17791921.4
(22) Anmeldetag: 18.10.2017
(51) Int. Cl.: A61M 5/20

(54) **ELEKTRONISCHES ZUSATZMODUL FÜR INJEKTIONSGERÄTE**
ELECTRONIC ADD-ON MODULE FOR INJECTION APPLIANCES
MODULE ÉLECTRONIQUE COMPLÉMENTAIRE POUR APPAREILS D'INJECTION

(30) Priorität: 09.11.2016 CH 14852016
(43) Veröffentlichungstag der Anmeldung: 18.09.2019
(73) Patentinhaber: Ypsomed AG, 3401 Burgdorf (CH)
(72) Erfinder: STREIT, Ursina, 3322 Schönbühl (CH); URBANEK, Leos, 3012 Bern (CH); GENTZ, Michael, 3400 Burgdorf (CH); RIHS, Jonas, 2552 Orpund (CH); RYTZ, Bernhard, 3436 Zollbrück (CH); FRUTIGER, Dominic, 8050 Zürich (CH)
(74) Vertreter: Meier Obertüfer, Jürg
(86) Internationale Anmeldenummer: PCT/CH2017/050006
(87) Internationale Veröffentlichungsnummer: WO 2018/085952

(56) Entgegenhaltungen:
- EP-A1- 2 182 456
- WO-A1-2010/098927
- WO-A1-2015/136564
- US-A1- 2014 379 874

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf ein medizinisches Überwachungssystem mit einem portablen elektronischen Zusatzmodul zum Aufsetzen auf ein Injektionsgerät.

### HINTERGRUND

Die Patentanmeldung EP 2781230 beschreibt eine auch als Autoinjektor bezeichnete Injektionsvorrichtung zum automatischen Ausschütten einer medizinischen Substanz mittels einer vorgespannten Ausschütt- oder Injektionsfeder welche über eine Kolbenstange einen Stopfen in eine Spritze drückt. Die Bewegung des Stopfens bewirkt eine Ausschüttung oder Abgabe der Substanz durch eine Nadel an einem distalen Ende der Spritze. Optional kann die Ausschüttfeder oder ein weiteres Energiespeicherelement auch eine Einstechbewegung der Spritze relativ zu einem Gehäuse der Vorrichtung in distale Richtung automatisch ausführen. Die Injektionsvorrichtung umfasst weiter eine in einer Längsrichtung zwischen einer proximalen und einer distalen Position verschiebbare Nadelschutzhülse. Die Nadelschutzhülse ist mit einer Nadelschutzhülsenfeder als separatem Antriebselement gekoppelt, welche die Nadelschutzhülse nach erfolgter Substanzabgabe in die distale Position schiebt, in welcher sie die Nadel seitlich umgibt oder abschirmt. Ein bewegliches Anschlagelement als Rückkopplungsvorrichtung zur Erzeugung eines akustischen Signals nach Abgabe einer bestimmten Menge an Substanz wird durch die Nadelschutzhülsenfeder zu einem Anschlag hin beschleunigt. Eine zweite Rückkopplungsvorrichtung mit einem durch die Ausschüttfeder beschleunigten Anschlagelement signalisiert den Beginn der Substanzabgabe.

Die Patentanmeldung EP 2182456 beschreibt ein portables elektronisches Zusatzmodul zum Aufsetzen oder Aufschnappen auf ein Injektionsgerät und zur Kopplung an ein proximales Ende, insbesondere an einen Dosiseinstellknopf, des Injektionsgerätes. Ein bewegliches Kontaktelement in Form eine Kugel berührt im aufgesetzten Zustand des Zusatzmoduls eine stirnseitige Endfläche des Injektionsgerätes und ist an einen piezoelektrischen Sensor oder einen Drucksensor gekoppelt. Durch den Sensor werden axiale Vibrationen detektiert, und durch eine Auswerteelektronik im Zusatzmodul werden charakteristische betriebliche Zustände oder Vorgänge identifiziert. Das Zusatzmodul hat eine geringe Ausdehnung in einer Längsrichtung des Injektionsgerätes, so dass letzteres auch bei aufgesetztem Zusatzmodul vom Benutzer direkt gehalten wird.

Der Begriff "Medikament" oder "medizinische Substanz" umfasst in diesem Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

### DARSTELLUNG DER ERFINDUNG

Es ist eine Aufgabe der Erfindung, eine einfache und kostengünstige Möglichkeit zur Überwachung oder Kontrolle auf korrekte Ausführung eines mit einem automatischen Injektionsgerät durchgeführten Injektionsvorgangs und zur Weiterleitung der injektionsrelevanten Daten zu schaffen. Es ist eine weitere Aufgabe der Erfindung, ein sicheres Zusammenwirken von Komponenten, Geräten und Systemen zur Erzeugung, Sammlung, und Verteilung von Daten im Zusammenhang mit dem Einsatz von Injektionsgeräten zu ermöglichen.

Diese Aufgabe wird gelöst mit Hilfe eines elektronischen Zusatzmoduls welches vor Injektionsbeginn auf ein automatisches Injektionsgerät mit einer Längsachse, welche ein proximales Ende und ein distales oder einstechseitiges Ende des Injektionsgerätes verbindet, lösbar aufgesetzt oder aufgesteckt wird. Erfindungsgemäss umfasst das Zusatzmodul einen Kraftsensor zur Messung einer durch das Injektionsgerät auf das aufgesetzte Zusatzmodul während eines Injektionsvorgangs ausgeübten oder übertragenen zeitveränderlichen axialen Kraftkomponente in Richtung der Längsachse. Erfindungsgemäss umfasst das Zusatzmodul einen Griff oder eine bevorzugte Griffposition zum Ergreifen und Halten des Injektionsgerätes und des aufgesetzten Zusatzmoduls, beziehungsweise des Zusatzmoduls und des eingesetzten Injektionsgerätes. Der Griff kann ein zur Ergreifung durch eine Hand eines Benutzers des Injektionsgerätes ergonomisch vorgeformter Teil eines Modulgehäuses des Zusatzmoduls sein, so dass alle weiteren im gekoppelten Zustand sichtbaren Bereiche des Zusatzmoduls und des Injektionsgerätes einem Benutzer als deutlich weniger geeignet zur Ergreifung erscheinen. Ein Benutzer welcher das Injektionsgerät ausschließlich über den Griff des aufgesetzten Zusatzmoduls ergreift kann keine Kräfte direkt auf das Injektionsgerät einleiten oder aus dem Injektionsgerät ausleiten. Insbesondere werden im statischen Gleichgewicht vergleichbare Axialkräfte an der Einstechstelle und über das Zusatzmodul auf das Injektionsgerät übertragen, so dass der Kraftsensor im Zusatzmodul nicht nur ein Vibrationsverhalten des Injektionsgerätes detektieren sondern auch eine konstante oder nur langsam veränderliche, keine Beschleunigung erzeugende axiale Krafteinleitung durch den Benutzer messen kann.

Das Zusatzmodul umfasst ein hülsenförmiges Modulgehäuse mit einer Geräteaufnahme oder Öffnung, in welche das Injektionsgerät vor Injektionsbeginn eingesetzt wird und welche im aufgesetzten Zustand das Injektionsgerät zumindest teilweise umgibt. Der Griff ist ebenfalls Teil des Modulgehäuses und umgibt die Geräteaufnahme, zudem weist der Griff in Richtung der Längsachse eine Ausdehnung von mindestens einer halben Handbreite eines Benutzers auf, insbesondere eine Ausdehnung von mindestens fünf oder mindestens acht cm. Der Griff ist also radial ausserhalb der Geräteaufnahme vorgesehen, was zu einer kompakteren Form führt als ein axial oder seitlich über das Injektionsgerät hinausreichender Ansatz am Zusatzmodul. Eine lichte Weite der Geräteaufnahme ist zumindest im Bereich des Griffs etwas grösser als ein Aussendurchmesser des Injektionsgerätes, so dass auch bei maximalem Griffdruck die Geräteaufnahme nicht auf das Injektionsgerät gepresst wird und keine Reibungs- oder Haltekräfte in Längsrichtung über die Geräteaufnahme zwischen Injektionsgerät und Zusatzmodul, unter Umgehung des Kraftsensors, übertragen werden.

In einer weiter bevorzugten Ausführungsform weist das Zusatzmodul einen lösbaren Haltemechanismus auf, welcher im aufgesetzten Zustand eine Bewegung des Zusatzmoduls relativ zum Injektionsgerät in Richtung der Längsachse einschränkt oder gar verunmöglicht. Durch eine Begrenzung der Relativbewegung in axialer Richtung können die während der Injektion auf den Kraftsensor in proximaler Richtung einwirkenden Kräfte begrenzt werden. Durch den Haltemechanismus sind Injektionsgerät und Zusatzmodul gegen unbeabsichtigtes Entkoppeln gesichert, und die bei einer Nadelschutzkappenentfernung in distaler Richtung auf das Zusatzmodul wirkenden Kraftspitzen können über das Zusatzmodul abgeleitet werden.

Das Zusatzmodul weist eine Auswerteelektronik auf, konfiguriert zur Identifikation von Vorgängen im, oder von Zuständen des, Injektionsgerätes während eines Injektionsvorganges, basierend auf Messungen des Kraftsensors, insbesondere basierend auf deutlichen Änderungen der Axialkraft hervorgerufen durch Bewegungen des Benutzers oder durch einen freigegebenen Antriebsmechanismus. Beispielsweise kann die Detektion eines Anstiegs der gemessenen axialen Kraftkomponente auf einen während einer gewissen Minimalinjektionszeit eine minimale Haltekraft nicht unterschreitenden Wert und eines nachfolgenden Abfalls der Axialkraft auf null von der Auswerteelektronik als Kraftaufwand eines Benutzers zwischen Einstechvorgang und Injektionsgeräteentfernung mit entsprechender Bewegung einer Nadelschutzhülse samt Druckbeaufschlagung einer zugehörigen Nadelschutzhülsenfeder identifiziert werden. Weiter können überlagernde Bewegungen von Komponenten des Injektionsgerätes, wie ein Drehen einer Hülse oder ein Auslenken eines Schnappers zur Verriegelung oder Entriegelung zu einem identifizierbaren, zeitlich begrenzten Anstieg oder Abfall in der gemessenen Axialkraft führen.

In einer Weiterentwicklung dieser vorteilhaften Variante werden auch kurzzeitige Kraftstösse von wenigen Millisekunden Dauer und resultierend aus automatischen, federgetriebenen Bewegungen eines Anschlagelementes des Injektionsgerät zur Signalisierung eines Ausschüttbeginns oder eines Ausschüttendes registriert oder festgestellt. Die gemessenen Kraftstösse können beispielsweise rasch abklingende Schwingungen oder Vibrationen des Injektionsgerätes relativ zum Zusatzmodul entsprechen. Insbesondere wird ein Beginn der Ausschüttung durch eine korrelierte Messung eines schnellen Anstiegs der Axialkraft auf einen Haltekraftwert und eines deutlichen Kraftstosses identifiziert, während ein Ende der Ausschüttung durch Messung eines Kraftstoss in Kombination mit einer konstanten axialen Haltekraft identifiziert wird. Bevorzugt umfasst die Signalisierung eine ungebremste Beschleunigungsphase des Anschlagelementes und einen nachfolgenden Anschlag oder Aufprall des Anschlagelementes zur Anregung von Schwingung im Injektionsgerät, welche primär durch den Benutzer als akustisches oder taktiles Klick-Signal wahrgenommen werden können und zusätzlich auch durch einen geeignet platzierten Axialkraftsensor messbar sind.

In einer vorteilhaften Weiterentwicklung wird ein kurzzeitiges Kraftstosssignal des Kraftsensors gefiltert und in einem Komparator oder einer Vergleichsschaltung mit einem ersten und bevorzugt einem zweiten oder weiteren Schwellwert verglichen und in ein mehrwertiges, insbesondere zwei- oder dreiwertiges diskretes Ereignismuster im Ausgangssignal des Komparators überführt. Stimmt ein derart ermitteltes Ereignismuster mit einem vorbestimmten und gespeicherten Basismuster überein, welches ein bestimmtes Basisereignis charakterisiert, wird das ursprüngliche Kraftstosssignal als zu dem bestimmten Basisereignis gehörig identifiziert. Es wird also ausgenutzt, dass ein Basisereignis, also beispielsweise ein Anschlag eines Anschlagelementes zur Signalisierung eines Ausschüttbeginns oder eines Ausschüttendes, über ein charakteristisches mehrwertiges Basismuster im Axialkraft-Ausgangssignal des Komparators verfügt, welches sich in jedem Ereignismuster eines einmalig registrierten Anschlagsereignisses wiederfindet.

In einer alternativen Weiterentwicklung der vorteilhaften Variante weist das Zusatzmodul ein Mikrofon oder akustischen Sensor auf zur Messung eines akustischen Signals während des Injektionsvorganges, insbesondere von durch die Luft übertragenen Schwingungen des Injektionsgerätes. Die Auswerteelektronik identifiziert in diesem Fall das Ereignis basierend auf Messungen des Mikrofons und Messungen des Kraftsensors. Beispielsweise wird ein Beginn der Ausschüttung durch eine korrelierte Messung eines schnellen Anstiegs der Axialkraft auf einen Haltekraftwert und eines deutlichen akustischen Signals identifiziert, während ein Ende der Ausschüttung durch Messung eines deutlichen akustischen Signals in Kombination mit einer konstanten axialen Haltekraft bestimmt wird.

Als Alternativen zum Mikrofon können auch ein einachsiger oder ein mehrachsiger Beschleunigungssensor oder ein Gyroskop eingesetzt werden. Bei diesen Alternativen wie auch beim Mikrofon ist die Platzierung des Sensors im Zusatzmodul gegenüber dem auf eine Kontaktfläche zum Injektionsgerät angewiesenen Kraftsensor weniger kritisch. In diesen Weiterentwicklungen wird also durch das Zusatzmodul einerseits eine axiale, insbesondere konstante, Mindestkraft des Benutzers auf das Injektionsgerät kontinuierlich gemessen, und andererseits eine zweite Messung mit einem von dem Kraftsensor verschiedenen Sensortyp durchgeführt, wobei die beiden Messungen gemeinsam zur Ereignisidentifikation ausgewertet werden.

Ein Zustand des Injektionsgerätes oder ein Prozess eines Injektionsvorganges kann also vom Zusatzmodul allein auf der Basis der Messungen des Axialkraftsensors, optional ergänzt durch Messungen eines zweiten Sensors, ermittelt werden. Tatsächlich ist eine aufwändige Detektion einer eingestellten oder ausgeschütteten Dosis für eine aussagekräftige Zustands- oder Prozessermittlung vielfach weder möglich noch erforderlich. Ein Injektionsvorgang kann auf Basis der drei Ereignisse Einstechen/Beginn Ausschüttung, Ende Ausschüttung, und Geräteentfernung als korrekt abgelaufen eingestuft werden. Sollte die Reihenfolge dieser Ereignisse oder eine Zeitspanne dazwischen nicht den Erwartungen entsprechen kann vom Zusatzmodul eine entsprechende Meldung erzeugt werden.

Ein Zusatzmodul nach der Erfindung kann in vorteilhafter Weise wiederholt eingesetzt werden zur Überwachung eines Gebrauchs oder Einsatzes von automatischen Einweg-Injektionsgeräten oder Autoinjektoren. Insbesondere ist das Zusatzmodul geeignet für einen Retrofit-Einsatz bei existierenden Injektionsgeräten, welche für eine Anpassung oder Modifikation nicht zur Verfügung stehen. In dieser Konfiguration sind im Injektionsgerät keine Sensoren vorgesehen zur Erfassung oder Bearbeitung von Sensordaten über die Operation des Gerätes, ebenso wenig eine Kommunikationsschnittstelle zur Übermittlung dieser Daten an einen Empfänger. Dementsprechend müssen die Kraftsensoren im Zusatzgerät derart ausgestaltet und positioniert werden, dass sie Zustandsänderungen oder Primärsignale von innerhalb des Injektionsgerätes feststellen können.

In einer weiteren Ausführungsform der Erfindung umfasst das Zusatzmodul eine Kommunikationseinheit zur drahtlosen Kommunikation mit einem mobilen Gerät, beispielsweise einem Mobiltelefon oder Smartphone, und/oder einen optischen, akustischen, oder taktilen Zustandsanzeiger. Ein angezeigter Zustand oder Status des Systems kann einen Gerätezustand des Injektionsgerätes, einen Modulzustand des Zusatzmoduls, oder einen Vorgangszustand eines laufenden oder abgeschlossenen Injektionsvorganges umfassen. Der Zustandsanzeiger kann einfach gehalten werden und sich auf einige wenige LEDs beispielsweise in Ampelfarben und/oder einen akustischen Signalgenerator zur Erzeugung von sprachunabhängigen Geräuschen oder Melodien beschränken. Dies ist insbesondere im Zusammenwirken mit den fortgeschrittenen graphischen Anzeigemöglichkeiten und Sprachausgabemöglichkeiten eines Smartphones vorteilhaft, da das drahtlos an das Zusatzmodul gekoppelte Smartphone die verfeinerte, über eine Statusanzeige hinausgehende Kommunikation mit dem Benutzer übernimmt. Die Zustandsinformation kann eine Angabe zum Ablauf einer Haltezeit oder Wartezeit umfassen, welche der Benutzer nach abgeschlossener Ausschüttung abwarten muss bevor das Injektionsgerät von der Injektionsstelle sicher entfernt werden kann. Eine einfache Erfassung der seit dem festgestellten Ausschüttende verflossenen Zeit und ein Vergleich mit einer Solldauer erlaubt es dem Benutzer den Zeitpunkt zum sicheren Entfernen des Injektionsgerätes anzeigen. Dies kann sowohl durch ein Zusatzmodul mit Zeiterfassungsfunktion als auch durch ein mit Echtzeit-Ereignisübertragung an das Zusatzmodul gekoppeltes mobiles Gerät erfolgen.

Für den Fachmann sind direkt und offensichtlich weitere Ausführungsformen und Ausgestaltungen erkennbar, welche sich aus Kombinationen der beschriebenen Beispiele oder Kombinationen der beschriebenen Beispiele mit dem allgemeinen Fachwissen des Fachmanns ergeben.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
Fig.1 zeigt ein Injektionssystem mit einem Injektionsgerät und einem Zusatzmodul;
Fig.2 zeigt eine Injektionsvorrichtung im Längsschnitt;
Fig.3 zeigt eine Explosionsdarstellung eines Zusatzmoduls;
Fig.4 zeigt gemessene Signale eines Kraftsensors und eines Mikrofons im Zusatzmodul;
Fig.5 zeigt ein Schema für eine Auswerteelektronik eines Zusatzmoduls;
Fig.6 zeigt ein durch einen Kraftsensor gemessenes und gefiltertes Signal;
Fig.7 zeigt eine Ereignismusterbildung aus einem Kraftstosssignal; und
Fig.8 zeigt ein erweitertes medizinisches Überwachungssystem.

### FIGURENBESCHREIBUNG

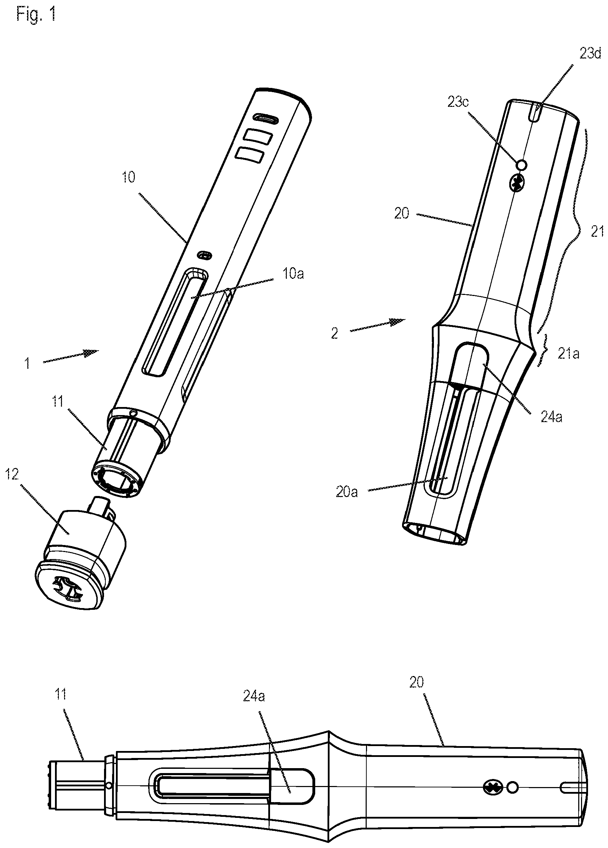

Fig.1 zeigt ein Injektionssystem mit einem Injektionsgerät 1 und einem Zusatzmodul 2, sowohl in getrenntem (Fig.1 oben) als auch in zusammengefügtem oder gekoppeltem (Fig.1 unten) Zustand. Das Injektionsgerät weist ein längliches, um eine Längsachse symmetrisches Gerätegehäuse 10 auf, und eine Nadelschutzhülse 11 welche zwischen einer ersten, eine Nadel des Injektionsgerätes abschirmenden Position (in Fig.1 gezeigt) und einer zweiten, die Nadel freigebenden Position verschiebbar ist. Ein Nadelschutzkappenentferner 12 ist im Auslieferungszustand auf ein distales Ende des Injektionsgerätes montiert und muss vor der Injektion zusammen mit einer die Nadel mechanisch schützenden Nadelschutzkappe entfernt oder abgezogen werden. Das Zusatzmodul hat ein längliches, hülsenförmiges Modulgehäuse 20 mit einem Hohlraum als Geräteaufnahme für das Injektionsgerät, welche an eine äussere Form des Gerätegehäuses abgestimmt ist, so dass zur Kopplung das Injektionsgerät in das Zusatzmodul eingeschoben werden kann. Das Zusatzmodul weist einen Löseknopf einer Haltevorrichtung 24a, eine optische Zustandsanzeige 23c, 23d für eine visuelle Rückmeldung an den Benutzer, und seitliche Öffnungen 20a auf, welche im gekoppelten Zustand mit Fenstern 10a im Gerätegehäuse ausgerichtet sind und den Blick auf eine im Injektionsgerät gelagerte Substanz ermöglichen. Alternativ kann das Zusatzmodul auch deutlich kürzer sein als das Injektionsgerät, also nur rund zwei Drittel oder gut halb so lang. Das Modulgehäuse weist auf der Aussenseite einen Griff 21 mit einer leicht konkaven Griffposition auf, welcher als distale Griffbegrenzung eine leichte Erhöhung 21a oder einen Bereich mit vergrössertem Durchmesser umfasst zur erleichterten Aufnahme einer Benutzerkraft in distale Richtung.

Zu Beginn einer Injektion wird das distale Ende des Injektionsgerätes, also die Nadelschutzhülse, durch den Benutzer auf die Einstechstelle gedrückt so dass die Nadelschutzhülse unter Spannung der Nadelschutzhülsenfeder in proximale Richtung in das Injektionsgerät eingeschoben wird und gleichzeitig die Nadel in die Einstechstelle eindringt. Um sicherzustellen dass in jedem Fall der Benutzer die Kraft während dem gesamten Injektionsvorgang inklusive Auslösung und Durchführung der Ausschüttung mittels Zusatzmodul auf das Injektionsgerät überträgt ist das Zusatzmodul als Hülse über einen Großteil des Injektionsgerätes ausgebildet. Das Injektionsgerät kann damit nicht unbeabsichtigt gehalten werden. Eine lichte Weite oder ein Innendurchmesser der Geräteaufnahme ist zumindest im Bereich des Griffs um einen über den Fertigungstoleranzen liegenden Betrag von wenigen Zehntels Millimeter grösser als ein Aussendurchmesser des Injektionsgerätes. Das Zusatzmodul weist radial also etwas Spiel zum Injektionsgerät auf, damit diese sich reibungsfrei zueinander bewegen können und die gesamte Kraft über den Kraftsensor übertragen wird.

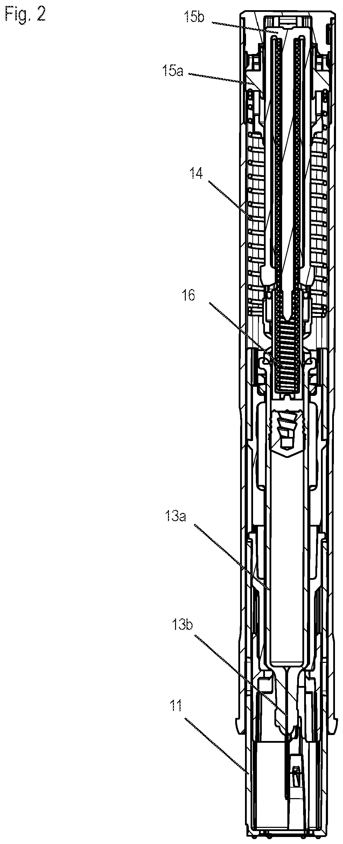

Fig.2 zeigt eine Injektionsvorrichtung im Längsschnitt, in einem Zustand vor der Injektion, in welche eine mit einer abzugebenden Substanz gefüllte Spritze 13a mit Nadel 13b eingesetzt ist. Eine hohlzylindrische Nadelschutzhülse 11 steht mit einem proximalen Ende mit einer rigiden Verriegelungshülse in Kontakt, welche wiederum an einer Nadelschutzhülsenfeder 14 anliegt. Am proximalen Ende der Nadelschutzhülsenfeder ist ein erstes Anschlagelement oder Klickelement 15a zur Markierung eines Ausschüttendes (End of Injection, Eol) vorgesehen. Eine Kolbenstange ist zu Beginn der Ausschüttung mittels Nocken mit dem ersten Anschlagelement verbunden, so dass das erste Anschlagelement zu Beginn der Ausschüttung um einen kleinen Hub nach vorne bewegt werden kann und dadurch die Nadelschutzhülsenfeder weiter gespannt oder komprimiert wird. Nach vollständiger Ausschüttung der Substanz und abgeschlossener Vorwärtsbewegung der Kolbenstange wird das erste Anschlagelement freigegeben und durch die Nadelschutzhülsenfeder proximal nach hinten beschleunigt, wo es auf eine Endkappe aufschlägt und ein für den Benutzer deutlich hörbares Klickgeräusch und einen spürbaren Schlag erzeugt. Ein zweites Anschlagelement 15b oder Klickelement ist zur Markierung eines Ausschüttbeginns (Start of Injection, Sol) vorgesehen. Ein Klick Pin ist anfänglich mit der Kolbenstange gegen eine Relativbewegung verriegelt. Bei Auslösung der Ausschüttung durch proximale Verschiebung der Nadelschutzhülse und der Verriegelungshülse erfolgt eine Freigabe von Kolbenstange und Klick-Pin, welche durch eine vorgespannte Ausschüttfeder 16 auseinandergetrieben werden. Diese Bewegung des Klick-Pins wird auf das zweite Anschlagelement übertragen, so dass das proximale Ende des zweiten Anschlagelements auf die Endkappe aufschlägt und ein deutlich hörbares Klickgeräusch und einen Kraftstoss oder Impuls erzeugt.

Alternativ zum gleichzeitigen Nadelschutzhülseneinschub und Nadeleinstechen kann eine im Injektionsgerät schiebbar gelagerte Spritze, welche im Auslieferungszustand inklusive Nadelspitze vollständig vom Gerätegehäuse umfasst ist, durch die Kraft einer Einstech- und/oder Ausschüttfeder relativ zum Gerätegehäuse bewegt werden. Diese automatisierte Einstechbewegung wird nach einem teilweisen oder vollständigen Einschieben der Nadelschutzhülse in das Injektionsgerät ausgelöst, ein Aufprall der beschleunigten Spritze auf ein Anschlagelement bewirkt ebenfalls einen deutlichen Kraftstoss gefolgt von einer annähernd konstanten Halte- oder Ausschüttkraft. Die vorhergehende Einschubbewegung der Nadelschutzhülse mit gleichzeitigem Spannen der Nadelschutzhülsenfeder kann dabei langsam erfolgen, im Falle einer vorgespannten Nadelschutzhülsenfeder möglicherweise erst nach Überwindung einer Federvorspannkraft. Die Bewegung kann einen kontinuierlichen Anstieg der gemessenen Axialkraft zur Folge haben, eventuell unter Aufwendung einer im Kraftverlauf sichtbaren und der Federkompressionskraft überlagerten minimalen Entriegelungs- oder Injektionssperrkraft. In einer weiteren Ausführungsform kann die Einstechbewegung auch durch einen vom Benutzer zu betätigenden Auslöseknopf gestartet werden.

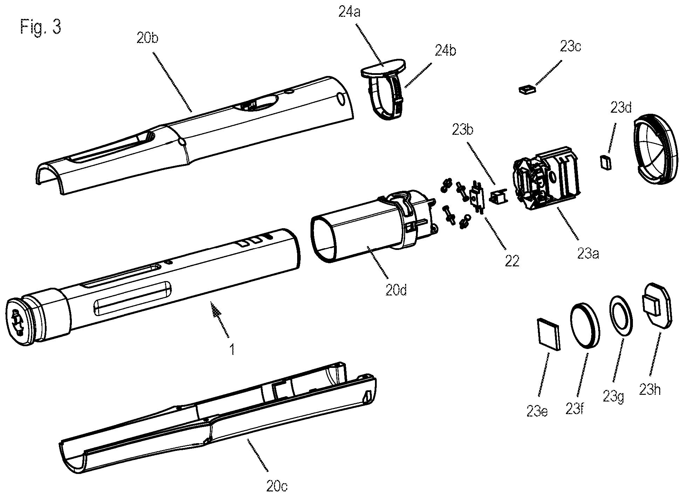

Fig.3 zeigt eine Explosionsdarstellung eines Zusatzmoduls gemäss der Erfindung. Das Modulgehäuse umfasst eine erste Gehäusehälfte 20b und eine zweite Gehäusehälfte 20c welche auf eine Modulhülse 20d aufgeschnappt sind. In einem Elektronikhalter 23a im hinteren Teil des Zusatzmoduls ist die Modulelektronik untergebracht, umfassend einen Kraftsensor 22 oder einen Drucksensor, einen Kontaktschalter 23b zur Detektion eines vollständigen Einschubs des Injektionsgeräts in das Zusatzmodul, eine Verbindungsanzeige 23c zur Signalisierung eines erfolgreichen Verbindungsaufbaus mit einem mobilen Gerät, eine Zustandsanzeige 23d mit LEDs und Lichtleitern in Form einer transparenten distalen Abschlusskappe. Weiter vorgesehen sind ein Mikrofon 23e, eine Batterie 23f als Energiequelle, ein Summer oder Lautsprecher 23g zur Erzeugung von Geräuschen, und eine Kommunikationseinheit 23h mit einem Bluetooth Low Energy (BLE) Chip inklusive Auswerteeinheit zur Signalverarbeitung und Datenspeicherung. Die Zustandsanzeige kann insbesondere folgende Zustände darstellen: Zusatzmodul aufgesteckt, Zusatzmodul bereit, Injektion läuft, Injektion beendet, Haltezeit abgelaufen, Zusatzmodul entfernen, BLE Zustand. Die Zustandsanzeige kann auch Warnungen ausgeben, wie Zusatzmodul falsch montiert, Zusatzmodul schaltet wegen Inaktivität aus, Batteriestand tief, Erinnerung um Zusatzmodul zu entfernen, oder gar Fehlermeldungen wie Injektionsbeendung während der Ausschüttung, Injektionsbeendung während der Haltezeit.

Eine Haltevorrichtung oder ein Haltemechanismus umfasst ein in der Modulhülse geführtes ringförmiges Übertragungselement 24b, eine fest am Übertragungselement angeordnete Haltestruktur oder Nase, eine Einrastfeder welche das Übertragungselement mit der Haltestruktur in eine Halteposition bewegt, und ein Löseknopf als Betätigungselement 24a zur Bewegung des Übertragungselements und zur Lösung der Haltestruktur aus der Halteposition. Die Haltestruktur ist in der Halteposition in eine Vertiefung wie etwa eine Kerbe oder ein Schlitz des Gehäuses des Injektionsgerätes eingerastet. Diese Vertiefung kann auch für andere Zwecke nutzbar sein und ist idealerweise in bestehenden Injektionsgeräten bereits vorhanden, wodurch am Injektionsgerät zum Einsatz des Zusatzmoduls keine Anpassung vorgenommen werden muss. Alternativ zur Druckbeaufschlagung des Löseknopfs und einer Bewegung des Übertragungselements senkrecht zur Längsachse kann das Betätigungselement auch eine schiebende Bewegung ausführen. Bei gelöster Haltestruktur kann das Injektionsgerät aus dem Zusatzmodul in axialer Richtung herausgleiten, der Haltemechanismus ermöglicht also ein lösbares Aufschnappen des Zusatzmoduls auf das Injektionsgerät. Das Modulgehäuse in Fig.3 unterscheidet sich von demjenigen in Fig.1, insbesondere durch die Anordnung der Haltevorrichtung innerhalb des Griffbereichs anstatt zwischen Griffbereich und distalem Gehäuseende. Die Haltevorrichtung wird also vom Benutzer beim Halten mit umfasst, der Löseknopf ist in diesem Falle etwas im Modulgehäuse versenkt, um ein unbeabsichtigtes Lösen durch die haltende Hand des Benutzers zu verhindern.

Die Haltevorrichtung kann durch entsprechende Ausbildung von Haltestruktur und Vertiefung in axialer Richtung etwas Spiel aufweisen, so dass sich der Kraftsensor von der Endkappe oder Kontaktfläche weg bewegen kann. In diesem Fall fehlt eine Vorspannung des Kraftsensors wenn das Injektionsgerät nicht auf die Injektionsstelle aufgesetzt und die Nadelschutzhülsenfeder nicht komprimiert ist. Entsprechend kann ein Signal oder Klick am Ende der Ausschüttung nach vorzeitiger Entfernung des Injektionsgerätes möglicherweise durch den Kraftsensor nicht mehr erkannt werden, so dass nur eine akustische Identifikation über das Mikrofon zur Vervollständigung des fehlerhaften Injektionsvorgangs beitragen kann. Andererseits kann durch kraftschlüssiges Einschnappen der Haltestruktur des Zusatzmoduls in die Vertiefung des Injektionsgerätes der Kraftsensor auch vorbelastet oder vorgespannt sein. In diesem Fall ist eine Vorspannstrecke im Modulgehäuse zwischen Haltevorrichtung und Kraftsensor entsprechend auf Zug vorbelastet, und unter Umständen sind erst externe Axialkräfte über einem gewissen Vorspannwert am Kraftsensor beobachtbar. So misst ein an der proximalen Stirnfläche des Injektionsgeräts anliegender Kraftsensor initial den Vorspannwert sowie jede beliebig kleine Benutzerkraft, welche unter Umgehung der Vorspannstrecke beispielsweise nach hinten abgeleitet wird. Andererseits wird bei der bevorzugten Anordnung des Griffs als Teil des die Geräteaufnahme umgebenden Modulgehäuses ein Teil der Axialkraft des Benutzers zumindest teilweise über die Vorspannstrecke auf den Kraftsensor geleitet. Dadurch wird zuerst die Zugbelastung in der Vorspannstrecke reduziert, und nur Benutzerkräfte oberhalb eines die Vorspannkraft nicht überschreitenden Minimalwertes werden durch den Kraftsensor zusätzlich zur Vorspannkraft gemessen.

In der gezeigten Ausführungsform kommt der Axialkraftsensor im aufgesetzten Zustand des Zusatzmoduls am proximalen, oder hinteren, Ende des Injektionsgerätes zu liegen. Er berührt also eine proximale Stirnfläche oder Endkappe des Injektionsgerätes, was gegenüber einer Kraftübertragung über eine seitlich am Gerät vorzusehende Kontaktfläche senkrecht zur Achse, also beispielsweise über eine proximale Ringfläche eines umlaufenden Flansches oder eines Abschnittes davon, eine Vereinfachung darstellt. Ebenso ist eine Umleitung der Axialkraft mittels beweglicher Komponenten am Zusatzmodul in eine von der Längsrichtung abweichende Richtung denkbar, etwa für eine nicht-axiale Platzierung des Kraftsensors.

Geeignete Kraftsensoren basieren auf einem piezoresistiven Effekt, also auf einer Änderung des spezifischen elektrischen Widerstandes eines Leiters bei mechanischer Verformung. Bei Kraftsensoren der FSS Serie des Anbieters Honeywell erhöht sich ein Widerstand von in einem Silizium-Messelement integrierten Piezowiderständen, wenn diese durch Kraftaufwand gebogen werden, was über eine Widerstandsbrücke messbar ist. Der Sensor überträgt eine angewendete Kraft zwischen null und 20 Newton über eine Edelstahlkugel direkt auf das Silizium-Messelement, wobei die Kugel um einen Hub von maximal 50 Mikrometer bewegt wird. Alternative Kraftsensoren zur Messung von Haltekräften umfassen auf Biegebalken angebrachte Dehnungsmessstreifen, oder FSR (Force Sensing Resistor) Drucksensoren.

Die Auswerteeinheit kann in der Kommunikationseinheit 23h integriert sein oder als eigenständiger Chip auf dem Elektronikhalter vorgesehen sein. Die Auswerteeinheit verarbeitet die Signale des Kraftsensors und der allenfalls vorgesehenen weiteren Sensoren, besorgt eine Zeitstempelung, und bestimmt aus den verarbeiteten Signalen und den zugehörigen Zeitstempeln eine konsolidierte Injektionsinformation. Letztere umfasst zumindest die Information dass eine Injektion zu einem bestimmten Zeitpunkt erfolgreich durchgeführt worden ist, oder welche Fehlhandlungen möglicherweise vorgenommen worden sind. Diese Information wird in einer Speichereinheit im Zusatzmodul gespeichert und/oder über die Kommunikationseinheit weitergeleitet. Die Speichereinheit kann auch Identifikationsdaten des Benutzers oder des Zusatzmoduls speichern.

Zu Energiesparzwecken kann der Kontaktschalter 23b auch als Aktivierungselement fungieren zur Aktivierung des Zusatzmoduls aus einem Stromsparmodus. Zusätzlich oder alternativ kann die Energiequelle wiederaufladbar sein, wozu das Zusatzmodul über einen geeigneten Stecker verfügt oder zur induktiven Energieübertragung von einer Ladestation vorgesehen ist. Die Ladestation kann ausschliesslich das Zusatzmodul aufnehmen, oder das Zusatzmodul mit eingesetztem Injektionsgerät, und kann auch von anderen aufladbaren Geräten des Benutzers mitbenutzt werden. Eine intelligente Ladestation kann als Basisstation fungieren und zusätzliche Funktionalitäten aufweisen, zum Beispiel eine Temperaturmessung des aufgenommenen Injektionsgerätes beziehungsweise des darin enthaltenen Medikamentes. Wird das Injektionsgerät nach einer Lagerung im Kühlschrank und vor dem Gebrauch im Ladegerät platziert kann eine solche Temperaturmessung am Injektionsgerät ein Signal ausgeben sobald eine Minimaltemperatur zur Verabreichung des Medikamentes erreicht ist. Während diesem Aufwärmvorgang kann auch die Energiequelle des Zusatzmoduls wiederaufgeladen werden, ohne weitere Intervention des Benutzers. Über eine weitere Kommunikationsschnittstelle kann die intelligente Ladestation kann auch Zeit und Datumsinformation zur Verfügung stellen zur Synchronisierung einer Uhr des Zusatzmoduls, oder als Rückfallebene zum mobilen Gerät wie unten beschrieben die konsolidierten Injektionsinformationen übernehmen.

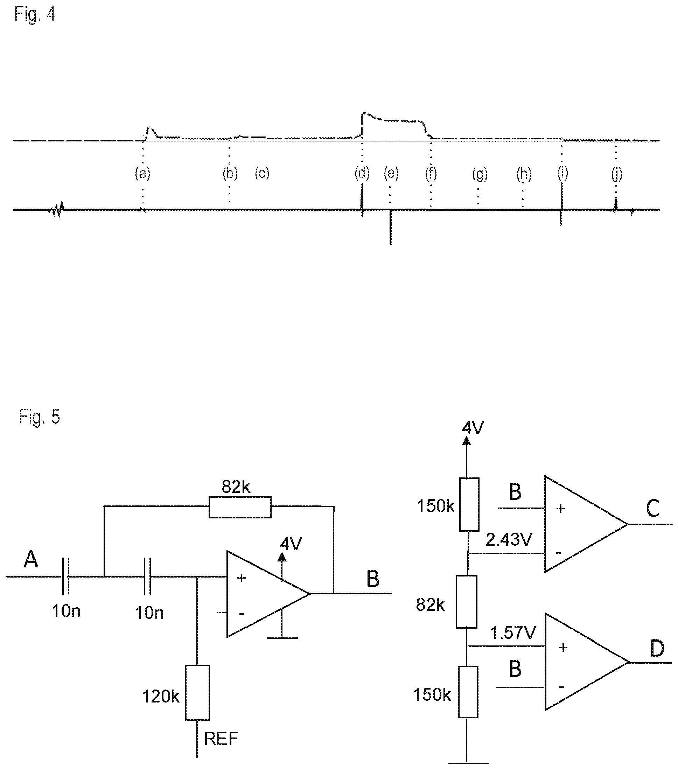

Fig.4 zeigt die während eines kompletten Injektionsvorganges von 20 Sekunden Dauer gemessenen Signale eines piezoresistiven Kraftsensors (oben) und eines Elektret Mikrofons (unten). Folgende Ereignisse sind den Signalen zugeordnet: (a) Verbinden von Zusatzmodul und Injektionsgerät, (b) Entfernen der Nadelschutzkappe vom Injektionsgerät, (c) Injektionsgerät zur Injektionsstelle bewegen/kippen, (d) Start der Ausschüttung (Start of Injection, Sol), (e) Ende der Ausschüttung (End of Injection, Eol), (f) Entfernen des Injektionsgeräts von der Injektionsstelle, (g) Injektionsgerät in eine horizontale Lage bringen, (h) Injektionsgerät ablegen, (i) Injektionsgerät aufheben und fallen lassen, (j) Injektionsgerät aus Zusatzmodul entfernen. Der Kraftabfall der gemessenen Axialkraft beim Entfernen oder Abheben des Injektionsgerätes von der Injektionsstelle (Ereignis f) ist kontinuierlicher als beim Einstechen (Ereignis d) und folgt der sich entspannenden Nadelschutzhülsenfeder, eventuell unter Ausbildung eines die Flanke überlagernden Peaks hervorgerufen durch eine Bewegung eines Nadelschutzhülsenverriegelungsmechanismus.

Zustände, Vorgänge und Ereignisse im Injektionsgerät und während des Injektionsvorganges können durch kombinatorisches Verketten der Informationen des Kraftsensors und des Mikrofons exemplarisch wir folgt identifiziert werden. Zur Detektion eines Startklicks wird die Kombination eines deutlichen akustischen Peaks und eines Kraftanstiegs (mit Verbleib auf dem Kräfteplateau) gefordert. Um einen Endklick zu detektieren wird die Kombination eines deutlichen akustischen Peaks und einer konstant hoch bleibenden Kraft verlangt. Das Entfernen des Injektionsgerätes samt Zusatzmodul von der Injektionsstelle wird durch den Kraftabfall nach dem Endklick detektiert. Wird das Injektionsgerät vor dem Endklick oder auch während der geforderten Haltezeit von der Injektionsstelle entfernt, kann dies durch den vorzeitigen Kraftabfall detektiert werden.

Fig.5 zeigt ein Schema für eine Auswerteelektronik als Teil der Auswerteeinheit, anzuwenden auf ein durch den Kraftsensor gemessenes Eingangssignals A. In einem Hochpassfilter mit einer Grenzfrequenz von 200 Hz (Fig.5, links) werden die niederfrequenten Signalanteile ausgefiltert. Das gefilterte Signal umfasst sowohl positive wie negative Ausschläge, entsprechend den Abweichungen im Kraftstosssignal gegenüber einem Haltekraftplateau. Um negative Spannungswerte zu vermeiden wird das Ausgangssignal des Filters auf einen Offset von 2V (halbe Versorgungsspannung) angehoben. Das resultierende Signal B wird einem Komparator (Fig.5, rechts) zugeführt, welcher das analoge Signal mit einem oberen und einem unteren Grenzwert eines Amplitudenfensters vergleicht und in Impulse umwandelt. Falls das derart präparierte Signal den oberen Grenzwert überschreitet wird ein erster Komparatorausgangswert C, beispielsweise ein Impuls von plus 1 V ausgegeben oder ein entsprechendes Bit oder Zeichen gesetzt. Falls das Signal den unteren Grenzwert unterschreitet wird ein zweiter Komparatorausgangswert D, beispielsweise ein Impuls von minus 1 V generiert. In der Folge wird ein dreiwertiges diskretes Ereignismuster als eine Folge unterschiedlich lange andauernder positiver und negativer Impulse, getrennt durch einen Neutral- oder Mittelwertpuls beliebiger Länge generiert.

Die Auswerteelektronik kann analog oder digital oder gemischt implementiert werden. Beispielsweise wird das hochpassgefilterte analoge Sensorsignal auf einen Mikroprozessor geführt, von diesem in ein digitales Signal umgewandelt und der digitalen, im Speicher des Mikroprozessors programmierten Komparatorfunktion zugeführt. Eine Abtastrate für die Digitalisierung des Kraftstosssignals muss gemäss Nyquist gewählt werden, für ein Kraftstosssignal entsprechend einem akustischen Klickgeräusch mit einem Frequenzspektrum bis 5 kHz ist also eine Abtastung alle 20 Mikrosekunden angebracht.

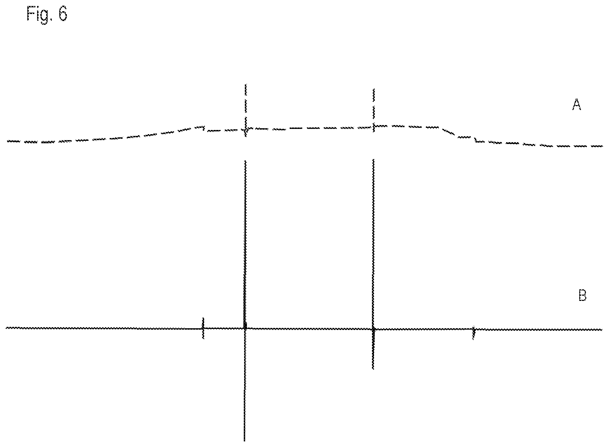

Fig.6 zeigt ein durch einen Kraftsensor gemessenes Eingangssignal A am Eingang eines Filters, sowie das durch Filterung des Signals A erzeugte gefilterte Signal B am Eingang eines Komparators. Der gesamte abgebildete Vorgang dauert 5 Sekunden, entsprechend sind die Kraftstosssignale zu Beginn und am Ende der Ausschüttung entsprechend einem Start-of-Injection und einem End-of-injection Klick nur als schmale Peaks erkennbar.

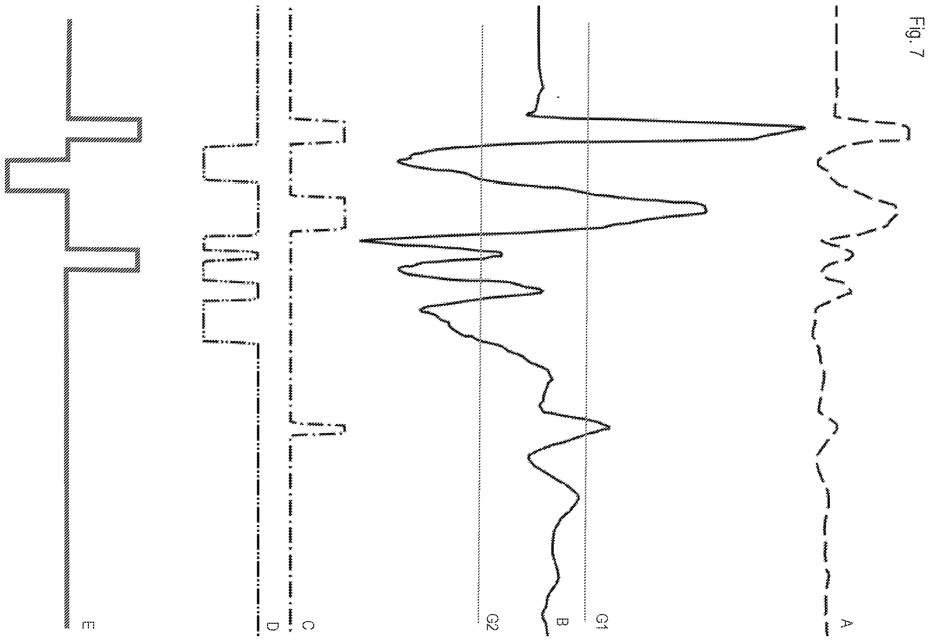

Fig.7 zeigt ein Beispiel für eine Ereignismusterbildung für das Kraftstosssignal zu Beginn der Ausschüttung in Fig.6, zeitlich aufgelöst über eine Dauer von 8 Millisekunden. Das ungefilterte Signal A und das gefilterte Signal B zeigen entsprechend mehr Struktur. Durch Vergleich des Signals B mit einem durch die obere horizontale Linie G1 dargestellten ersten Grenzwert oder Schwellwert wird ein erstes Komparatorausgangssignal C generiert mit drei Pulsen unterschiedlicher Länge. Durch Vergleich des Signals B mit einem durch die untere horizontale Linie G2 dargestellten zweiten Grenzwert oder Schwellwert wird ein zweites Komparatorausgangssignal D generiert, hier umfassend eine Folge von vier Pulsen unterschiedlicher Länge. Die Folge der zweiten Impulse ist hier der besseren Lesbarkeit halber mit negativen Werten dargestellt. Das aus dem selektierten Kraftstoss resultierende Ereignismuster mit den beiden Komponenten C und D stimmt mit dem charakteristischen Basismuster oder der Signatur eines Startklicks, dargestellt durch die Kurve E, insofern überein als dass die drei Pulse des Basismusters zeitlich vollständig innerhalb von Pulsen des Ereignismusters liegen. Dementsprechend wird das Kraftstosssignal als von einem Startklick herrührend identifiziert. Die Bestimmung der Grenzwerte G und des zugehörigen Basismusters E erfolgt bevorzugt durch ein iteratives Vorgehen mit wiederholtem Aufzeichnen und Analysieren von Kraftstössen mit bekanntem Ursprung. Die selektierten Grenzwerte führen dann idealerweise zu einer minimalen Streuung in den Ereignismustern und somit zu einer hohen Reproduzierbarkeit. Der vorgestellte Algorithmus kann erfolgreich vorgängige Aktionen und Fehlmanipulationen wie Aufheben des Zusatzmoduls, Montage des Zusatzmoduls, Entfernen des Nadelschutzes, drehen, fallen lassen, laute Umgebungsgeräusche, zittriges Halten als nicht injektionsrelevante, mit keinem Basissignal übereinstimmende Kraftstösse identifizieren oder ausschliessen.

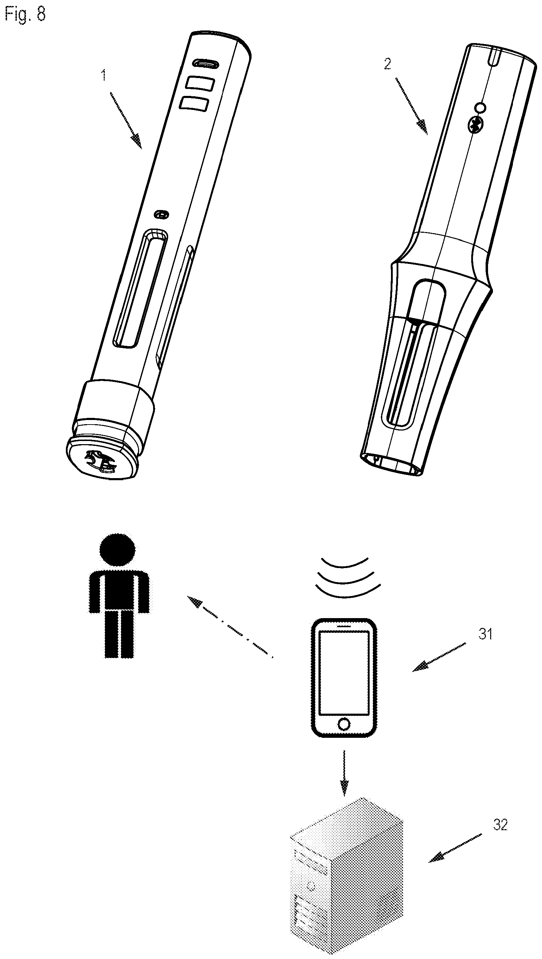

Fig.8 zeigt ein erweitertes medizinisches Überwachungssystem mit einem Injektionsgerät 1, einem Zusatzmodul 2, einem mobilen Anzeige- und Ausgabegerät 31 welches mit dem Zusatzmodul drahtlos kommunizieren kann, und einem optionalen stationären, auf de-lokalisierten Datenspeichern (Cloud-Computing) basierenden Datenverarbeitungssystem oder Expertensystem 32. Das Zusatzmodul überträgt Daten eines Injektionsprozesses und/oder konsolidierte Injektionsinformationen drahtlos an ein kompatibles mobiles Gerät, beispielsweise ein schlaues Mobiltelefon (Smartphone) mit einer speziellen Applikation, oder einen entsprechend konfigurierten tragbaren Computer.

Das mobile Gerät muss initial eingerichtet und konfiguriert werden, etwa durch installieren einer Applikation und Registrierung des Benutzers. Dies kann durch eine Patientendatenkarte geschehen, welche über Nahfeldradiokommunikation (NFC) oder optische QR Codes alle relevanten Daten auf das mobile Gerät überspielt. Falls das mobile Gerät sich in Reichweite des Zusatzmoduls befindet kann diese Datenübertragung während einem Injektionsvorgang in Echtzeit erfolgen oder erst nach dessen Beendigung. Im erstgenannten Fall kann das mobile Gerät in Echtzeit Instruktionen an den Benutzer abgeben und diesen so durch die nächsten Schritte führen. Allenfalls können die Injektionsinformationen auch auf dem Zusatzmodul gespeichert werden und erst später, in konsolidierter Form, übertragen werden. Die vom mobilen Gerät empfangenen Daten können durch den Benutzer ergänzt werden, etwa durch Angabe der Injektionsstelle, und werden auf geeignetem Weg an das Expertensystem weitergegeben. Letzteres speichert die Daten und versorgt Patienten, medizinisches Personal, und Krankenversicherer mit gezielten Informationen und unterstützt so eine Befolgung eines Therapieplans durch den Benutzer des Injektionsgerätes.

### BEZUGSZEICHENLISTE

- 1: Injektionsgerät
- 10: Gerätegehäuse
- 10a: Fenster
- 11: Nadelschutzhülse
- 12: Nadelschutzkappenentferner
- 13a: Spritze
- 13b: Nadel
- 14: Nadelschutzhülsenfeder
- 15a, 15b: Anschlagelement
- 16: Ausschüttfeder
- 2: Zusatzmodul
- 20: Modulgehäuse
- 20a: Öffnung
- 20b, 20c: Gehäusehälften
- 20d: Modulhülse
- 21: Griff
- 21a: Griffbegrenzung
- 22: Kraftsensor
- 23a: Elektronikhalter
- 23b: Kontaktschalter
- 23c: Verbindungsanzeige
- 23d: Zustandsanzeige
- 23e: Mikrofon
- 23f: Batterie
- 23g: Summer
- 23h: Kommunikationseinheit
- 24a: Löseknopf
- 24b: Übertragungselement
- 31: Mobiles Gerät
- 32: Datenverarbeitungssystem

## Patentansprüche

1. Injektionssystem mit einem Injektionsgerät (1) umfassend
- eine in einer Längsrichtung zwischen einer proximalen und einer distalen Position verschiebbare Nadelschutzhülse (11);
- eine Nadelschutzhülsenfeder (14), welche mit der Nadelschutzhülse (11) gekoppelt ist und welche die Nadelschutzhülse (11) nach erfolgter Substanzabgabe in die distale Position schiebt, in welcher sie die Nadel seitlich umgibt oder abschirmt, wobei die Nadelschutzhülsenfeder (14) während einem Einstechvorgang gespannt wird;
das Injektionssystem darüber hinaus umfassend ein Zusatzmodul (2) zum Aufsetzen auf das Injektionsgerät (1) mit einer Längsachse, das Zusatzmodul (2) umfassend
- einen Kraftsensor (22) zur Messung einer durch das Injektionsgerät auf das aufgesetzte Zusatzmodul während eines Injektionsvorgangs ausgeübten Kraftkomponente in Richtung der Längsachse,
- eine Auswerteelektronik zur Auswertung von Messungen des Kraftsensors (22) zwecks Identifikation eines Vorganges oder eines Zustandes des Injektionsgerätes (1) **dadurch gekennzeichnet, dass** das Zusatzmodul (2) konfiguriert ist zur Identifikation eines Einstechvorgangs aus einem Anstieg einer durch den Kraftsensor gemessenen Axialkraft auf einen zumindest annähernd konstanten und durch die komprimierte Nadelschutzhülsenfeder bedingten Wert und wobei das Zusatzmodul (2) ein hülsenförmiges Modulgehäuse mit einer Geräteaufnahme zur Aufnahme des Injektionsgerätes und einen Griff (21) zum Halten des Injektionsgerätes und des aufgesetzten Zusatzmoduls aufweist, wobei eine lichte Weite der Geräteaufnahme im Bereich des Griffs grösser als ein Aussendurchmesser des Injektionsgerätes (1) ist, so dass bei maximalem Griffdruck die Geräteaufnahme nicht auf das Injektionsgerät gepresst wird und keine Reibungs- oder Haltekräfte in Längsrichtung über die Geräteaufnahme zwischen Injektionsgerät (1) und Zusatzmodul (2), unter Umgehung des Kraftsensors (22), übertragen werden können.

2. Injektionssystem nach Anspruch 1, **dadurch gekennzeichnet**, das die Geräteaufnahme das Injektionsgerät im aufgesetzten Zustand zumindest teilweise umgibt, wobei der Griff die Geräteaufnahme umgibt und in Richtung der Längsachse eine Ausdehnung von mindestens einer halben Handbreite eines Benutzers aufweist.

3. Injektionssystem nach Anspruch 2, **dadurch gekennzeichnet, dass** das Zusatzmodul einen Haltemechanismus aufweist, welcher das Injektionsgerät im aufgesetzten Zustand in Richtung der Längsachse fixiert.

4. Injektionssystem nach einem der Ansprüche 1 bis 3, **dadurch gekennzeichnet, dass** die Auswerteelektronik konfiguriert ist zur Identifikation einer Bewegung eines Anschlagelementes (15a, 15b) des Injektionsgerätes, insbesondere einer den Beginn oder das Ende einer Ausschüttung signalisierenden Anschlagbewegung des Anschlagelementes.

5. Injektionssystem nach Anspruch 4, **dadurch gekennzeichnet, dass** die Auswerteelektronik konfiguriert ist zur Erzeugung eines mehrwertigen Ereignismusters aus einem gemessenen Kraftstosssignal und zum Vergleich des erzeugten mehrwertigen Ereignismusters mit einem Basismuster.

6. Injektionssystem nach einem der Ansprüche 1 bis 3, **dadurch gekennzeichnet, dass** das Zusatzmodul ein Mikrofon (23e) aufweist zur Messung eines akustischen Signals des Injektionsvorganges, und dass die Auswerteelektronik konfiguriert ist zur Identifikation, basierend auf Messungen des Mikrofons, einer den Beginn oder das Ende einer Ausschüttung signalisierenden Bewegung eines Anschlagelementes des Injektionsgerätes.

7. Injektionssystem nach einem der Ansprüche 1 bis 3, **dadurch gekennzeichnet, dass** das Zusatzmodul einen Beschleunigungssensor aufweist, wobei die Auswerteelektronik konfiguriert ist Messungen vom Beschleunigungssensor und vom Kraftsensor zur Ereignisidentifikation gemeinsam auszuwerten.

8. Injektionssystem nach einem der vorhergehenden Ansprüche, **dadurch gekennzeichnet, dass** das Zusatzmodul eine drahtlose Kommunikationseinheit (23h) zur Kommunikation mit einem mobilen Gerät und/oder eine Zustandsanzeige (23d) zur Anzeige eines Zustandes des Injektionsgerätes umfasst.

9. Injektionssystem nach einem der vorhergehenden Ansprüche, wobei die Nadelschutzhülsenfeder am Ende der Ausschüttung ein Anschlagselement beschleunigt, **dadurch gekennzeichnet, dass** das Zusatzmodul konfiguriert ist zur Identifikation eines Anschlags des beschleunigten Anschlagselementes aus einem durch den Kraftsensor detektierten Kraftstoss.

## Claims

1. Injection system having an injection device (1), comprising
- a needle protection sleeve (11) which is displaceable in a longitudinal direction between a proximal and a distal position;
- a needle protection sleeve spring (14) which is coupled to the needle protection sleeve (11) and which, after substance delivery has taken place, pushes the needle protection sleeve (11) into the distal position, in which it laterally surrounds or shields the needle, the needle protection sleeve spring (14) being tensioned during a piercing process;
the injection system further comprising an add-on module (2) for attaching to the injection device (1), having a longitudinal axis, the add-on module (2) comprising
- a force sensor (22) for measuring a force component, in the direction of the longitudinal axis, that is exerted by the injection device on the attached add-on module during an injection process;
- an evaluation electronics system for evaluating measurements of the force sensor (22) for the purpose of identifying a process or a state of the injection device (1),
**characterized in that** the add-on module (2) is designed to identify a piercing process from an increase in an axial force measured by the force sensor to an at least approximately constant value caused by the compressed needle protection sleeve spring, and
the add-on module (2) having a sleeve-shaped module housing having a device receptacle for receiving the injection device and a handle (21) for holding the injection device and the attached add-on module, a clear width of the device receptacle in the region of the handle being greater than an outer diameter of the injection device (1), so that, at maximum grip pressure, the device receptacle is not pressed onto the injection device and no friction or retaining forces can be transmitted in the longitudinal direction via the device receptacle between the injection device (1) and the add-on module (2) while bypassing the force sensor (22).

2. Injection system according to claim 1, **characterized in that** the device receptacle at least partially surrounds the injection device in the attached state, the handle surrounding the device receptacle and having an extension of at least half a hand width of a user in the direction of the longitudinal axis.

3. Injection system according to claim 2, **characterized in that** the add-on module has a holding mechanism which secures the injection device in the direction of the longitudinal axis in the attached state.

4. Injection system according to any of claims 1 to 3, **characterized in that** the evaluation electronics system is designed to identify a movement of a stop element (15a, 15b) of the injection device, in particular a stop movement of the stop element which signals the start or the end of a discharge.

5. Injection system according to claim 4, **characterized in that** the evaluation electronics system is designed to generate a multivalent event pattern from a measured force impact signal and to compare the generated multivalent event pattern with a base pattern.

6. Injection system according to any of claims 1 to 3, **characterized in that** the add-on module has a microphone (23e) for measuring an acoustic signal of the injection process, and **in that** the evaluation electronics system is designed to identify, on the basis of measurements of the microphone, a movement of a stop element of the injection device which signals the start or the end of a discharge.

7. Injection system according to any of claims 1 to 3, **characterized in that** the add-on module has an acceleration sensor, the evaluation electronics system being designed to conjointly evaluate measurements from the acceleration sensor and from the force sensor for event identification.

8. Injection system according to any of the preceding claims,
**characterized in that** the add-on module comprises a wireless communication unit (23h) for communicating with a mobile device, and/or a status display (23d) for displaying a state of the injection device.

9. Injection system according to any of the preceding claims, the needle protection sleeve spring accelerating a stop element at the end of the discharge, **characterized in that** the add-on module is designed to identify a stop of the accelerated stop element from a force impact detected by the force sensor.

## Revendications

1. Système d'injection comportant un appareil d'injection (1) comprenant
- un manchon de protection d'aiguille (11) pouvant être déplacé dans une direction longitudinale entre une position proximale et une position distale ;
- un ressort de manchon de protection d'aiguille (14) qui est accouplé au manchon de protection d'aiguille (11) et qui, après que la substance a été administrée, pousse le manchon de protection d'aiguille (11) dans la position distale, dans laquelle il entoure ou protège l'aiguille latéralement, dans lequel le ressort de manchon de protection d'aiguille (14) est tendu pendant un processus de pénétration ;
le système d'injection comprenant en outre un module supplémentaire (2) destiné à être mis en place sur l'appareil d'injection (1) avec un axe longitudinal, le module supplémentaire (2) comprenant
- un capteur de force (22) permettant de mesurer une composante de force dans la direction de l'axe longitudinal, laquelle composante de force est exercée par l'appareil d'injection sur le module supplémentaire mis en place pendant un processus d'injection ;
- une électronique d'évaluation permettant d'évaluer des mesures du capteur de force (22) en vue de l'identification d'un processus ou d'un état de l'appareil d'injection (1)
**caractérisé en ce que** le module supplémentaire (2) est configuré pour identifier un processus de pénétration à partir d'une augmentation d'une force axiale mesurée par le capteur de force jusqu'à une valeur au moins approximativement constante et liée au ressort de manchon de protection d'aiguille comprimé et
dans lequel le module supplémentaire (2) présente un boîtier de module en forme de manchon comportant un réceptacle pour appareil permettant de recevoir l'appareil d'injection et une poignée (21) permettant de maintenir l'appareil d'injection et le module supplémentaire mis en place, dans lequel une largeur intérieure du réceptacle pour appareil dans la zone de la poignée est supérieure à un diamètre extérieur de l'appareil d'injection (1), de sorte qu'à la pression maximale de la poignée, le réceptacle pour appareil n'est pas pressé contre l'appareil d'injection et aucune force de frottement ou de maintien ne peut être transmise dans la direction longitudinale par l'intermédiaire du réceptacle pour appareil entre l'appareil d'injection (1) et le module supplémentaire (2) en contournant le capteur de force (22).

2. Système d'injection selon la revendication 1, **caractérisé en ce que** le réceptacle pour appareil entoure au moins partiellement l'appareil d'injection à l'état mis en place, dans lequel la poignée entoure le réceptacle pour appareil et présente une extension d'au moins une demi-largeur de main d'un utilisateur dans la direction de l'axe longitudinal.

3. Système d'injection selon la revendication 2, **caractérisé en ce que** le module supplémentaire présente un mécanisme de maintien qui fixe l'appareil d'injection dans la direction de l'axe longitudinal à l'état mis en place.

4. Système d'injection selon l'une des revendications 1 à 3,
**caractérisé en ce que** l'électronique d'évaluation est configurée pour identifier un mouvement d'un élément de butée (15a, 15b) de l'appareil d'injection, en particulier un mouvement de butée de l'élément de butée signalant le début ou la fin d'une distribution.

5. Système d'injection selon la revendication 4, **caractérisé en ce que** l'électronique d'évaluation est configurée pour générer un motif d'événement à plusieurs valeurs à partir d'un signal de choc de force mesuré et pour comparer le motif d'événement à plusieurs valeurs généré avec un motif de base.

6. Système d'injection selon l'une des revendications 1 à 3,
**caractérisé en ce que** le module supplémentaire présente un microphone (23e) permettant de mesurer un signal acoustique du processus d'injection, **et en ce que** l'électronique d'évaluation est configurée pour identifier, sur la base de mesures du microphone, un mouvement d'un élément de butée de l'appareil d'injection signalant le début ou la fin d'une distribution.

7. Système d'injection selon l'une des revendications 1 à 3,
**caractérisé en ce que** le module supplémentaire présente un capteur d'accélération, dans lequel l'électronique d'évaluation est configurée pour évaluer conjointement des mesures du capteur d'accélération et du capteur de force pour l'identification d'événements.

8. Système d'injection selon l'une des revendications précédentes, **caractérisé en ce que** le module supplémentaire comprend une unité de communication sans fil (23h) permettant de communiquer avec un appareil mobile et/ou un moyen d'affichage d'état (23d) permettant d'afficher un état de l'appareil d'injection.

9. Système d'injection selon l'une des revendications précédentes, dans lequel le ressort de manchon de protection d'aiguille accélère un élément de butée à la fin de la distribution, **caractérisé en ce que** le module supplémentaire est configuré pour identifier une butée de l'élément de butée accéléré à partir d'un choc de force détecté par le capteur de force.
